# EUROPEAN PATENT APPLICATION

(11) **EP 4 825 450 A2**
(43) Date of publication of application: **21.10.2026**
(21) Application number: 26194551.3
(22) Date of filing: 10.05.2019
(51) Int. Cl.: A61Q 15/00

(54) **APPLICATOR AND SYSTEM FOR PHARMACEUTICAL PREPARATION AND METHOD OF USE**

(30) Priority: 11.05.2018 JP 2018092243; 11.05.2018 US 201862670475 P
(62) Document number(s) of the earlier application(s) in accordance with Art. 76 EPC: 19799730.7 / 3 790 425
(71) Applicant: Brickell Biotech, Inc., Boulder, CO 80301 (US)
(72) Inventor: BRINKMAN, Herbert, Boulder, 90301 (US); KOLENG, John, Boulder, 90301 (US); ALZENSTAT, Aron, Boulder, 90301 (US); SKLAWER, Andrew, Boulder, 90301 (US); NAGAO, Tatsuro, Shizuoka, 426-8646 (JP); TAKAOKA, Masayoshi, Shizuoka, 426-8646 (JP)
(74) Representative: Thurgood, Alexander John

(57) **Abstract**

An applicator and applicator system are provided for a topically or transdermally applying a pharmaceutical preparation or formulation. The applicator and applicator system can reduce drip during use, provide efficient transfer of a dose of the pharmaceutical preparation or formulation from the applicator to the skm of a patient, and can be used hands-free, which can reduce, prevent or eliminate cross-contamination to other areas, including undesired application to other parts of the skin or to other individuals. The applicator and applicator system include a therapeutic surface which can receive a single dose of the pharmaceutical preparation and can be manipulated by the user to transfer and apply the pharmaceutical preparation on a body surface.

## Description

### TECHNICAL FIELD

The present invention relates to an applicator and applicator system suitable for topical administration of a fluid pharmaceutical preparation having a moderate degree of viscosity.

### BACKGROUND

Conventionally, in order to apply a liquid or gel pharmaceutical preparation to an outer surface of a body, such as to the skin of a human, a bottle and pump is provided to deliver the preparation directly to the body, or into the hand for manually applying the preparation.

Applying certain preparations by hand can be disadvantageous for many reasons, including the "feel" of the preparation. For example, a preparation feeling tacky or slimy to the touch may be undesirable for manual application. Also, applying the preparation by hand may result in cross-contamination of areas of the body or other persons unintended to come into contact with the preparation. For example, topical testosterone preparations are contraindicated, and may be considered unsafe, for contact by females or children.

Various solutions to these inconveniences and disadvantages have been attempted. For example, deodorants have been formulated for direct application to the axilla from a roll-on applicator having a roller integrally formed or affixed to the container, thereby avoiding initial application of the deodorant to the hand. However, in the case of a higher viscosity formulation, the roller may become clogged or bound with the preparation, especially following one or more uses where drying of the unused preparation can occur between uses.

There are also brushes and sponge type applicators available. Although the preparation may not be touched directly by the hand using these applicators, residue of the preparation following one or more applications can build up causing the applicator to become less efficient for use, or cause a hygiene risk because the applicator is difficult or inconvenient to wash. A pharmaceutical preparation of moderate-to-high viscosity may also be more difficult to remove than solutions or creams.

Other types of applicators are also known in the art, and are described in, for example, International Patent Publications, Pub. Nos. WO 2008/083423, WO 2013/000778, and WO 2015/088848. International Publication No. WO 2008/083423 describes applying a low viscosity liquid preparation (300 centipoise or less at 25 °C) using an applicator having a concave surface disposed within an elastically deformable wall. According to the published Abstract of this application, the described implement is useful for applying a volume of liquid to a treatment surface. The implement includes a support means onto which is mounted a receptacle bounded by an elastically deformable wall, the receptacle and wall defining a reservoir space which receives the liquid. The receptacle and wall serve as a working surface used to spread the liquid over the treatment surface. The wall is resiliently deformable so that, in use, the working surface maintains contact with the treatment surface when spreading the liquid. The described implement has a specific application in applying a transdermal lotion to the axilla area of the user.

This applicator surface can be disadvantageous for use with moderate-to-high viscosity preparations because a certain amount of the preparation can remain in the reservoir of the applicator after application, resulting in administration of a lesser dose than is dispensed from the container.

WO 2013/000778 describes an applicator system for applying a viscous preparation to human skin and specifically is intended for administration of a high viscosity (3,000 centipoise or more at 25°C). The applicator system comprises a hard polypropylene, and convex therapeutic surface. According to the Abstract, this applicator system is useful for applying a viscous liquid to the human skin and comprises a metering dispenser, a container holding the viscous liquid, a pump for metering the liquid, and an applicator detachably connected to the dispenser. The applicator comprises a convex therapeutic surface for receiving the metered amount of the liquid from the dispenser, but does not comprise a peripheral ridge, such as the elastically deformable wall described in WO 2008/083423.

The applicator described in WO 2013/000778 is not particularly suitable for preparations having a moderate degree of viscosity and below, e.g., a viscosity below 3000 centipoise at 25°C. Preparations having moderate viscosity, or lower, are fluid enough to run off the convex applicator surface with no peripheral ridge to prevent the preparation from running off the surface. This, among other disadvantages, can make it difficult to apply a predetermined amount of the preparation to the affected area, especially when the preparation has a low-to-moderate viscosity (less than 3000 centipoises at 25°C.)

In WO 2015/088848, an applicator is described having a flexible membrane forming a folded wall structure that moves in the axial direction freely when pressure is applied to the membrane. According to the Abstract, the applicator for applying a fluid to a surface comprises a flexible membrane comprising a central opening, and comprising an inner wall and an outer wall at least partially surrounding the upper outer surface of the support, and a membrane holder for fixedly securing a lower end of the inner wall of the flexible membrane to the upper outer surface of the support. The upper surface of the membrane holder and the inner wall of the flexible membrane define a reservoir for holding a fluid, and wherein the lower end of the outer wall of the flexible membrane is free to move axially relative to the upper outer surface of the support when pressure is applied to the upper folded wall of the flexible membrane.

What is needed is an applicator and system suitable for local, topical or transdermal administration of pharmaceutical preparations having a moderate degree of viscosity.

### SUMMARY OF THE INVENTION

The subject invention concerns a novel applicator and applicator system for applying a pharmaceutical preparation or formulation to the body surface. The applicator can be part of the applicator system comprising a container for holding and storing a multi-dose volume of the pharmaceutical preparation, a dispensing means, such as a pump, to dispense one or more doses of the stored pharmaceutical preparation from inside the container to an area outside the container for use. A preferred embodiment comprises an applicator that can serve as a cap for the container and can optionally include a bottom cap serving as a base for the container. The pharmaceutical preparation is a fluid, and can be a solution, suspension, lotion, ointment, cream, foam or gel.

In a preferred embodiment, the applicator and applicator system are particularly useful for applying and topically or transdermally administering a metered dose of a pharmaceutical preparation. Preferably, the pharmaceutical preparation is a low to moderately viscous gel formulation. In a more preferred embodiment, the subject invention comprises an applicator or applicator system for applying a metered dose of an antiperspirant or hyperhidrosis medication to an afflicted area of the body such as the axilla.

According to one embodiment of the present invention, the applicator or applicator system is suitable for topical or transdermal administration of pharmaceutical formulations or preparations having a moderate degree of viscosity. A moderate degree of viscosity is generally understood as a viscosity of greater than about 300 centipoise at 25°C, but less than about 3,000 centipoise at 25°C. According to one embodiment of the present invention, the applicator comprises a top surface which is substantially flat for receiving a dispensed dose of the preparation from the container, which advantageously can provide greater than 90% delivery or transfer of the dispensed dose to the body surface. The substantially flat surface can further include a perimetric ridge or embossing which can facilitate retention of the preparation prior to administration, and further facilitate a more complete transfer of the preparation from the applicator surface to the body. The perimetric ridge or embossing can serve to retain the pharmaceutical preparation on the top surface of the applicator, including during administration, by preventing the low-to-moderately viscous preparation from being pushed over the edge of the top applicator surface during the process of applying the preparation to the body.

Also, according to an embodiment of the present invention, it is possible to provide an applicator system suitable for topical or transdermal administration formulations of sofpironium bromide and other compositions intended to treat hyperhidrosis.

The subject invention therefore comprises an applicator for applying a pharmaceutical composition to a skin surface of a patient in need thereof, the applicator comprising one or more substantially rigid side walls bounding a cavity which is open at a bottom end and closed at a top end by a top wall positioned perpendicularly to said one or more side wall(s) to form a cap. The applicator, or applicator cap, is configured to fit over and enclose a top portion of a container and dispenser for the pharmaceutical composition. The side walls are substantially rigid, meaning that they retain their general shape in their axial dimension (top to bottom) at all times, even when pressure is applied to the top or bottom of the wall, but have a thickness which is thin enough to provide, with manual pressure, slight malleability or deformation in their radial dimension (side-to-side) to facilitate and allow for detachable engagement of the applicator with a top portion of the container or dispenser, said closed top end of the cap having an outer surface comprising:
■ a central, substantially flat and continuous outer face which is solid and non-porous such that liquid cannot pass through said central outer face, the flat outer face being useful for receiving one or more doses of the pharmaceutical preparation dispensed thereon from the pharmaceutical preparation container, and
■ a peripheral ridge or embossing bounding the flat face and defining or forming a reservoir area for retaining the pharmaceutical preparation within said reservoir area on the flat outer central face when the pharmaceutical preparation is dispensed thereon.

The one or more substantially rigid side walls flexibly engage with and detachably affix to the top portion of the container or dispenser. The substantially rigid side wall or walls can comprise on their inner surface, and preferably at the bottom edge of the applicator, a ridge or protrusion formed thereon to matingly engage with the top portion of the container or the dispenser. By "bottom edge" of the applicator is meant relatively positioned nearer the open end of the applicator compared to its relative position with the top end of the applicator. The applicator side walls preferably matingly engage the top portion of the container or the dispenser and may form a substantially airtight seal. The side wall ridge or protrusion forming an airtight seal is not critical or required for the invention, but the tighter the seal, the better it prevents evaporation or drying of the pharmaceutical preparation within the container. The inner ridge or protrusion also functions to retain the applicator in close proximity to the container, which can be aesthetically preferred by consumers.

The applicator side wall ridge or protrusion can be formed as threads to threadingly engage the top portion of the container or the dispenser (forming a "screw-on'' or "twist-lock" configuration for the applicator) or can be a plurality of protrusions serving as hooks positioned intermittently, preferably equidistantly, around the inner face of the side wall (forming a "snap-on" configuration for the applicator). Either can also be configured as a child-proof attachment as is conventional in the art.

In a preferred embodiment, the side wall is a single circumferential wall forming a cross-sectionally circular or oval shaped applicator. The shape of the applicator is not critical so long as it conforms to and affixes fittingly with the container. So, for example, the applicator can comprise four side walls forming a cross-sectionally rectangular shaped applicator cap. The outer top surface of the top end of the applicator can comprise a ridge peripherally bounding the central flat top face of the applicator, and preferably the peripheral ridge has a top surface which is rounded. The ridge preferably is circumferential for a cross-sectionally circular or oval cap
In a more preferred embodiment, the one or more side walls of the applicator comprise indentations to facilitate gripping and handling for attaching or detaching the cap.

And in a further preferred embodiment, the applicator can comprise an overcap which covers at least the central flat surface of the applicator, or can cover the entire top surface of the applicator, including the outer peripheral ridge.

Advantageously, the central flat continuous face of the outer surface of the applicator can provide a reservoir for receiving the dispensed dose or doses of pharmaceutical preparation and can be useful for application of the pharmaceutical preparation to the skin surface of the patient. The peripheral ridge serves to retain the dispensed pharmaceutical preparation within the reservoir area formed in the central flat face during dose actuation and administration of the pharmaceutical preparation. The applicator can be especially useful for efficient application and transfer of the pharmaceutical preparation to an axilla of a user or patient being administered the pharmaceutical preparation. Preferably, the preparation is a pharmaceutical preparation comprising an active pharmaceutical ingredient useful to treat or ameliorate excessive sweating or hyperhidrosis, such as sofpironium bromide. The sofpironium bromide is preferably provided in a gel formulation having moderate viscosity.

The subject invention further concerns a system for applying a pharmaceutically acceptable composition to a skin surface of a patient in need thereof, the system comprising:
■ a container for housing and storing a plurality of doses of the pharmaceutical preparation, the container having an opening at a top end for receiving and engaging a dispenser, e.g., a pump dispenser, for dispensing a metered dose of the pharmaceutical preparation, and
■ an applicator covering the dispenser and detachably engaging the top portion of the container or a portion of the dispenser, said applicator comprising one or more substantially rigid side walls bounding a cavity which is open at a bottom end and closed at a top end by a top wall perpendicular to said one or more side wall, said closed top end of the cap having an outer surface comprising a central flat, continuous face, which is solid and non-porous such that liquid cannot pass through said face, and a peripheral ridge bounding the flat face and defining a reservoir area for retaining the pharmaceutical preparation within said reservoir area on the flat face when the pharmaceutical preparation is dispensed thereon.

Advantageously, the applicator for receiving and retaining the dispensed pharmaceutical preparation within the reservoir area of said flat face facilitates the administration and application of the pharmaceutical preparation to the patient from the flat face when applied to the skin surface of said patient.

The system can further comprise an overcap which engages with the cap and covers at least the central flat surface of the applicator. The container further comprises a bottom end which is open or in open communication with ambient air outside the container to allow equilibration of pressure within the container following dispensing of the pharmaceutical preparation from the container. The container can further comprise a piston within the container which reduces storage volume of contents within the container upon each dose dispensation and compresses the contents for ultimately dispensing substantially all the pharmaceutical preparation from the container. Alternatively, the container can comprise a collapsible inner liner disposed within the container which reduces storage volume of contents within the container upon each dose dispensation and compresses the contents for ultimately dispensing substantially all the pharmaceutical preparation from the container.

In a preferred embodiment, the container includes a bottom cap forming a base of the container. The system can comprise any dispenser but is preferably a pump dispenser for a moderately viscous pharmaceutical preparation, and more preferably a metered-dose pump dispenser.

In use, the applicator and applicator system of the subject invention includes a method for treating or ameliorating excessive sweating or hyperhidrosis in a patient in need thereof, said method comprising the steps of:
a) providing a container having a contents comprising a pharmaceutical preparation effective for treating or ameliorating excessive sweating or hyperhidrosis, and a dispenser engaged with said container for dispensing the pharmaceutical preparation from said container, said container including a detachable applicator fitting over a top portion of said container and dispenser, said applicator comprising one or more substantially rigid side walls bounding a cavity which is open at a bottom end and closed at a top end perpendicular to said one or more side wall, said closed top end of the applicator having an outer surface comprising a central flat and continuous face which is solid and non-porous, and a peripheral ridge bounding the flat face and forming a reservoir area for retaining the pharmaceutical preparation within said reservoir area on the flat face when the pharmaceutical preparation is dispensed thereon from the container; the applicator optionally comprising an overcap which covers at least the reservoir area on the flat face of the applicator;
b) removing the applicator from the container and, if present, removing the overcap from the applicator;
c) dispensing one or more doses of the pharmaceutical preparation into the reservoir area on the outer flat face of the applicator; and
d) applying the dispensed dose or doses onto the skin surface of the patient. The method can advantageously be carried out on the skin surface of the axilla of the patient.

The method of the invention can be preferably carried out using a pharmaceutical preparation provided in the form of a solution, lotion, cream, light ointment, foam or gel. A further preferred embodiment of the subject method comprises the pharmaceutical preparation being dispensed from the container by a metered dose dispenser as a single dose per axilla to which the preparation is administered.

The preferred method of the invention comprises the employment of a pharmaceutical preparation comprising an active pharmaceutical ingredient which is effective for treating or ameliorating excessive sweating or hyperhidrosis, and more preferably wherein the active pharmaceutical ingredient is sofpironium bromide. The active pharmaceutical ingredient used in the subject method can be formulated as a low-to-moderately viscous preparation comprising one or more carriers or excipients selected from the group consisting of a solvent, a co-solvent, a penetration enhancer, a pH-modifying agent, and a viscosity modulating agent, such as a gelling agent or thickener. In one preferred embodiment, the pharmaceutical preparation comprises isopropyl myristate.

In one preferred embodiment, the method of the invention comprises dispensing and administering the pharmaceutical preparation to the skin surface as needed or desired, and can be administered one or more times per week, or one or more times per day. One preferred us includes administering the pharmaceutical preparation at least one time per day. However, it would be understood that the method can be carried out multiple times per day, as needed, but for convenience of use, the method is typically carried out from one time per day to about four times per day. In another preferred embodiment, the method can comprise administration before the patient's sleep period. The application of the pharmaceutical preparation can be carried out immediately prior to the patient's sleep period, about one hour to about two hours prior to the patient's sleep period, or from about two hours to about four hours prior to the patient's sleep period.

The present invention includes the following embodiments:
[1] an applicator system for applying to the body surface, a topical or transdermal pharmaceutical preparation having a viscosity of 100 to 3000 centipoise at 25°C, preferably from about 100 to 2000 centipoise at 25°C, and more preferably from about 300 to 2000 centipoise at 25°C, and a container for holding the pharmaceutical preparation, the container and the applicator/cap to be detachably engaged with one another. The applicator/cap is configured to receive the pharmaceutical preparation, typically a single or metered dose, on its top surface, or therapeutic surface, such that the pharmaceutical preparation can be transferred to the body surface for effect or treatment. The top surface is formed of a substantially rigid inelastic material, and is a single flat, slightly concave, or slightly concave or slightly convex applicator system. In a preferred embodiment, the top outer face of the applicator is substantially flat. In another embodiment, the top outer face of the applicator can be slightly curved in a convex or concave configuration, wherein the angle of rise or fall is about 15°or less from the horizontal plane.
[2] In one embodiment, the pharmaceutical formulation preferably has a viscosity of about 100 to 1100 centipoise at 25°C, and is a formulation selected from the group consisting of liquids, gels, lotions, and creams.
[3] In an embodiment, the pharmaceutical formulation is preferably a preparation having a viscosity of about 100 to 900 centipoise at 25°C.
[4] In an embodiment, the pharmaceutical formulation is a preparation containing one or more C1-C4 alcohols of 40% (w/w) or more, being in an applicator system according to any one of embodiments [1] to [3].
[5] The amount of alcohol used in each dose being about 1 mL.
[6] The therapeutic surface is surrounded by the outer peripheral ridge. and the peripheral ridge portion leading to the side wall of the applicator.
[7] The top face of the applicator forms a single planar therapeutic surface which occupies more than 10% of the area of the top face of the applicator.
[8] Preferably, the flat therapeutic surface occupies more than 60% of the area of the top face of the applicator.
[9] In an embodiment where the therapeutic surface is concave or convex, the height difference between the highest part of the therapeutic surface and the lowest portion of the therapeutic surface is about 0.1 mm to 4 mm.
[10] More preferably, in an embodiment where the therapeutic surface is concave or convex, the height difference between the highest part of the therapeutic surface and the minimum portion of the therapeutic surface is about 0.1 mm to 1 mm.
[11] The therapeutic surface has a substantially circular planar shape and has a diameter ranging between about 25 mm and about 45 mm. For an embodiment of the applicator having a substantially elliptical shape, the therapeutic surface has a major axis of about 45 mm and a minor axis of about 25 mm.
[12] The container comprises a barrel portion for accommodating the pharmaceutical preparation, and being open at the top (having a top "mouth") to receive a pump that is mounted on the mouth of, and extends into the barrel portion of, the container.
[13] The therapeutic surface is configured to receive a single dose of the pharmaceutical preparation discharged from the pump by one or more actuations of the dispenser.
[14] The pharmaceutical formulation is a preparation containing sofpironium bromide used as a hyperhidrosis therapeutic agent.
[15] The applicator/cap having the therapeutic surface thereon, is a coating tool applied to and contacting the axilla for delivery, transfer or administration of the pharmaceutical preparation to the axilla for treatment.
[16] An applicator used for applying a pharmaceutical preparation for topical or transdermal administration on a body surface, the pharmaceutical preparation having a viscosity in a range from about 100 to 3000 centipoise at 25°C, the applicator including a therapeutic surface capable of receiving a single dose of the pharmaceutical preparation to be applied on a body surface, the therapeutic surface being formed by a rigid, non-elastic material, and forming a single recessed reservoir area.
[17] The applicator described in [16], in which the pharmaceutical preparation is selected from the group consisting of a gel agent, a lotion agent, a cream agent and a liquid agent, each having viscosity in a range from 100 to 1500 centipoise at 25°C.
[18] The applicator described in [17], in which the pharmaceutical preparation has viscosity in a range from 100 to 1000 centipoise at 25°C.
[19] The applicator described in any one of [16] to [18], in which the pharmaceutical formulation is a preparation containing 40w/w% or more of one or a plurality of C1-C4 alcohols.
[20] The applicator described in any one of [16] to [19], in which the single dose is in a range from 0.1 mL to 1 mL.
[21] The applicator described in any one of [16] to [20], in which the therapeutic surface includes only an outer peripheral ridge portion merging into a side wall of the applicator and a top surface, including the therapeutic surface surrounded by the outer peripheral ridge portion.
[22] The applicator described in [21], in which the top outer surface forms a single flat portion and the single flat portion includes a therapeutic surface that occupies 5% or more of a surface area of the applicator top surface.
[23] The applicator described in [22], in which the single flat portion occupies 60% or more of the surface area of the applicator top surface.
[24] The applicator described in any one of [16] to [23], in which a difference in height between an uppermost portion of the outer ridge and the therapeutic surface of the applicator is in a range from 0.1 mm to 4.0 mm.
[25] The applicator described in [24], in which the difference in height between the uppermost portion of outer ridge and the therapeutic surface of the outer ridge is in a range from 0.1 mm to 1.5 mm.
[26] The applicator described in any one of [16] to [25], in which the therapeutic surface has a substantially circular shape having a diameter in a range from 20 mm to 45 mm or a substantially oval shape having a long or short diameter in a range from 20 mm to 45 mm as viewed from above.
[27] The applicator described in any one of [16] to [26], in which the pharmaceutical preparation includes sofpironium bromide used for treatment of hyperhidrosis.
[28] The applicator described in any one of [16] to [27] in which the applicator is configured to be applied to an axilla.

Applicator system according to an embodiment of the present invention is an applicator system suitable for topical or transdermal administration of preparations having a low-to-moderate degree of viscosity. The preparation, without permitting the preparation to run off the applicator top surface, can be easily applied to the body surface. Also, after applying to the body surface, the preparation does not substantially remain in the applicator or on the applicator surface.

Therefore, the applicator system according to an embodiment of the present invention, for the users, is an applicator system that can be used conveniently and appropriately.

### BRIEF DESCRIPTION OF THE DRAWINGS

FIG. 1 is an external plan view of the applicator system according to an embodiment of the present invention.
FIG. 1A is an external view of the cover member or overcap.
FIG. 2 is an external elevational side view of the container and dispenser.
FIG. 3 is a side view of the applicator.
FIG. 4 is a top perspective view of the applicator.
FIG. 5 is a top plan view of the applicator.
FIG. 5A is a side cross-sectional view of the applicator, sectioned along the line a-a in Figure 5.
FIG. 6 is a partial enlarged view of FIG. 5A.
FIG. 7 is a perspective cross-sectional view of an enlarged portion of the therapeutic surface of the applicator.
FIG. 8 shows a partial side cross-sectional view of an alternative embodiment of the applicator/cap.
FIG. 9 shows partial perspective cross-sectional view of the applicator/cap illustrated in FIG. 8
FIG. 10 shows a partial side cross-sectional view of an alternative embodiment of the applicator/cap.
FIG. 11 shows a partial perspective cross-sectional view of the applicator/cap illustrated in FIG. 10.
FIG. 12 is a partial side cross-sectional view of an alternative embodiment of the applicator/cap.
FIG. 13 is a diagram illustrating a method of using the applicator system.
FIG. 14 is a diagram illustrating a method of using the coating tool by a patient.

The following is a list of reference symbols used in the Figures.

### REFERENCE LETTER/NUMBER LIST

- C: center axis
- L: horizontal distance
- L2: external diameter of side surface
- S, S1: area
- H: height difference
- 1: applicator system
- 3: container
- 3A: bottle portion
- 3B: dispenser or pump portion
- 5, 5A, 5B: applicator
- 5C: applicator (comparative example)
- 7, 47, 57: therapeutic surface
- 9: cover member or overcap
- 10: cavity formed by side and top wall of the applicator
- 11: applicator side wall
- 13: lower end portion of applicator
- 15: top wall of applicator
- 16: protrusions or hooks for detachably affixing the applicator to the container
- 17: side wall surface
- 18: side wall indentation
- 20: shoulder portion
- 21: gradient portion (undercut)
- 22, 42, 52: outer peripheral ridge portion
- 22A, 52A: top surface of outer or peripheral ridge
- 24, 44, 54: top outer, or therapeutic surface of applicator
- 42a: top surface of outer or peripheral ridge
- 42b: therapeutic surface

### DETAILED DESCRIPTION OF THE INVENTION

The terms "viscous degree," "viscosity degree," "consistency" or "consistency degree" used herein, all refer to and are synonymous with or used interchangeably with "viscosity," meaning the thickness or flowability of the fluid pharmaceutical preparation or formulation, which is typically measured in units termed "centipoise."

The terms "formulation", "preparation," "pharmaceutical formulation." and "pharmaceutical preparation" are used interchangeably and refer to a pharmaceutically acceptable preparation comprising an active ingredient and other ingredients, solvents, excipients, and the like, as described, and as understood in the pharmaceutical and medical arts.

Pharmaceutical preparations for topical or transdermal administration in the present invention may contain at least one or more active ingredients. Active ingredient may be a variety of physiologically active substance, but are not limited, for example, hyperhidrosis therapeutic agents, antifungal agents, antibacterial agents, hormone substitutes, analgesics, may be such as respiratory medicine.

Preferred active ingredient, for example, is disclosed as an active ingredient of the hyperhidrosis therapeutic agent in patent document 4, the formula (1) 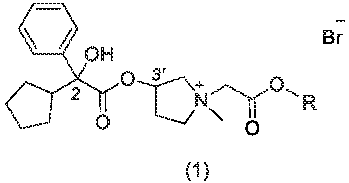 wherein, R and absolute arrangement is as defined in International Pub. No. WO 2015/138776 or International Publication No. WO 2017/015485, each of which is incorporated in its entirety by reference. That is, R is a methyl or ethyl, the compound is a compound represented by R in the second place and 1 ' and 3 ' position or having an R, S or RS solid-isomer arrangement, or a mixture thereof. Particularly preferably, the compound is 3'(R)-[2(R)-Cyclopentylphenyl-hydroxyacetoxy]-1'-methyl-1'-ethoxycarbonylmethyl-pyrrolidinium bromide, also referred to as "sofpironium bromide").

The "moderate degree of viscosity" in the present specification, is measured by the method to be described later herein, refers to the viscosity of greater than about 100 to less than about 3000 centipoise (25 °C), more narrowly, defines the degree of viscosity of 300 to 3000 centipoise (25 °C), and, further more narrowly, defines the degree of viscosity of 300 to 1100 centipoise (25 °C). The "low degree of viscosity" in the present specification, is measured by the method to be described later herein, refers to the viscosity of less than about 100 centipoise (25 °C) and, more broadly, defines the viscosity of less than about 300 centipoise (25 °C). The "high degree of viscosity" in the present specification, is measured by the method to be described later herein, refers to the viscosity of greater than about 3000 centipoise (25 °C).

Applicator system preferred viscosity of the present invention is low-to-moderate viscosity of less than about 3000 centipoise at 25°C. Ranges of viscosity for a pharmaceutical preparation used in connection with the applicator system of the invention include at a low end of the range from about 50, 100, 150, 200, 250, and 300 centipoise at 25°C and at the high end of the range from about greater than 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900 and less than 3000 centipoise of the range from about, including but not limited to ranges between about 100 to about 2000 centipoise (25°C), preferable ranges between about 100 to about 1500 centipoise (25°C), more preferable ranges between about 100 to about 1100 centipoise (25°C), further more preferable ranges between about 100 to about 900 centipoise (25°C), and particularly preferable ranges from about 400 to about 850 centipoise (25°C). A low viscosity ranges from about greater than zero to about 300 or less than 300 centipoise at 25°C and can range from about 10-300 centipoise at 25°C, from about 50 to about 300, centipoise at 25°C, from about 100 to about 300 centipoise at 25°C, from about 200 to about 300 centipoise at 25°C, from about 250 to about 300 centipoise at 25°C, from about 10 to about 250 centipoise at 25°C, from about 10 200 centipoise at 25°C, from about 50 to about 200 centipoise at 25°C, from about 50 to about 150 centipoise at 25°C or about 50 to about 100 centipoise at 25°C .

A medium or moderate viscosity can be greater than 300 up to about 3000 centipoise at 25°C and can range from about 300 to less than 3000 centipoise at 25°C, about 500-2500, about 1000 to about 2500 centipoise at 25°C, from about 1500-2500 centipoise at 25°C, from about 2000-2500 centipoise at 25°C, from about 300 to about 2500 centipoise at 25°C, from about 300 to about 2000, centipoise at 25°C, from about 300 to about 1500 centipoise at 25°C, from about 300 to about 1000 centipoise at 25°C or from about 500 to about 1000 centipoise at 25°C.

"Pharmaceutical formulations" or "pharmaceutical preparation" herein, may be a pharmaceutical formulation for topical or transdermal administration of low-to-moderate degree of viscosity, various solvents in addition to one or more active ingredients, additives, and can comprise a stabilizer, pharmaceutically acceptable excipients, or the like.

The pharmaceutical preparation for topical or transdermal administration suitable for the applicator and its applicator system according to one embodiment of the present invention is a preparation having viscosity of a medium or moderate degree and can be prepared by adding viscosity modifier as necessary.

As the "viscosity enhancer," "viscosity modifier," "viscosity agent," or "consistency modifier" in the specification, a thickener and/or a gelling agent can be used. The viscosity modifier is used for causing efficacy to be exerted by having the preparation held for a certain period of time on an applied portion on the body surface.

Specifically, any compatible or pharmaceutically acceptable cellulosic polymer or other well-known gelling agent, such as hydroxypropyl cellulose (HPC), hydroxypropyl methylcellulose (HPMC), carboxy vinyl polymer and the like may be used.

A solvent contained in the pharmaceutical preparation in the specification can be, but is not limited to, a C1-C4 alcohol, water, an oil base, a fatty acid ester and the like can be used. The "C1-C4 alcohol" in the specification refers to methanol, ethanol, n-propanol, isopropanol, n-butanol, isobutanol, sec-butanol, tert-butanol, ethylene glycol, propylene glycol, glycerin and the like. As the C1-C4 alcohol used for the pharmaceutical preparation applied to the applicator and its applicator system according to one embodiment of the present invention is not particularly limited as long as the viscosity of a low-to-medium or moderate degree can be obtained as the pharmaceutical preparation, but ethanol is the most preferable. Ethanol concentration in the preparation is preferably 40w/w% or more, more preferably 50w/w% or more and further preferably within a range from 60w/w% to 99w/w% and particularly preferably within a range from 70w/w% to 85w/w%.

Regarding the pharmaceutical preparation for topical or transdermal administration used in the present invention, the aforementioned viscosity modifier can be used as appropriate so that the viscosity of a low-to-medium or moderate degree of viscosity can be obtained. If an ethanol formulation with 0 to 15w/w% of sofpironium bromide in the whole preparation amount is to be prepared, the formulation having viscosity of a desired low to medium or moderate degree can be obtained by adding 1.25w/w% of HPC.

The "topical or transdermal administration" in the specification means to apply the pharmaceutical preparation on a target area of a human body surface or its peripheral portion (i.e., the area surrounding the target area).

The "body surface" in the specification refers to a skin surface of a human or other animal, preferably a mammal. Specifically, it refers to the skin surface of limbs, a body part, a head part and the like or more specifically, the skin surface of a palm, a face, a shoulder part, a chest part, a buttock part, an abdomen part, a back part, a genital part, an axilla or the like and nails and the like. According to one embodiment of the present invention, the body surface (applied portion) suitable for the application includes, but is not limited to, the axilla or any body surface requiring treatment, for example the palm of the hand, sole of the foot, or genital area, but preferably not mucosal surfaces such as the eye or inside the mouth.

According to one embodiment of the present invention, a "container" is a substantially hollow container bounding a cavity into which the pharmaceutical preparation having the viscosity of the low-to-medium or moderate degree is filled. The container including a pump attached to a mouth part is preferable so that an appropriate amount of the pharmaceutical preparation is received by an applicator. That is, a container including a bottle portion for accommodating the pharmaceutical preparation and a pump attached to the mouth part of the bottle portion is preferable.

According to one embodiment of the present invention, a preferable container is a container including a bottle portion with a substantially cylindrical shape having a substantially circular cross-sectional shape with a diameter from 30 mm to 50 mm or a substantially oval cylindrical shape having a substantially oval sectional shape with long or short diameter from 30 mm to 50 mm and a pump attached to a mouth part of the bottle porti on.

The "pump" in the specification refers to any commonly available or marketed dispensing mechanism, such as a negative pressure pump from which a single dose is dispensed by pushing down a dispensing part by fingers, palm and the like. This can be referred to as an actuation, or a "working" of the pump mechanism to dispense the preparation from the container.

According to one embodiment of the present invention, a preferable single dose (that is, a dispensed amount at one session) is 0.01 mL to 3.0 mL, more preferably 0.1 mL to 1.5 mL, further preferably 0.2 mL to 1.0 mL, furthermore preferably 0.5 mL to 0.8 mL, and particularly preferably 0.55 mL to 0.75 mL. The dispenser can be a metered dose dispenser that provides a measured and pre-determined dose with each dispensing action.

According to one embodiment of the present invention, the "applicator" is a tool for receiving a single dose of the preparation from the container and for applying it on the body surface. The applicator includes a therapeutic surface capable of applying the preparation on the body surface. By using the applicator, the preparation can be applied on the body surface easily and reliably without contaminating the hand or the like by the preparation.

According to one embodiment of the present invention, the "applicator system" includes the applicator of the present invention and indicates the set of materials suitable for medical use.

According to one embodiment of the present invention, the applicator system includes a container for accommodating the aforementioned pharmaceutical preparation and an applicator. Preferably, the applicator system includes aforementioned container and an applicator detachably engaging the container.

The applicator according to one embodiment of the present invention is detachably connected to the container and is separated from the container before use. After the single dose of the preparation is supplied from the container by the dispenser, and to the therapeutic surface of the separated applicator, the therapeutic surface is pressed onto and in contact with the body surface of a human, and the preparation is spread over an area of the body surface. Separation of the applicator from the container and reattachment/reconnection after the use can be carried out easily. Therefore, when not in use, the applicator and container are stored connected until the next use.

A part of the container to which the applicator is attached is not particularly limited. However, the applicator is preferably attached so as to cover the mouth part of the container, for example. The applicator is particularly preferably attached so as to cover an upper part of the container including a dispensing port of the pump and the like.

According to one embodiment of the present invention of an applicator, the applicator is not detachably attached to the container.

According to one embodiment of the present invention, the "therapeutic surface" is an area in a certain range on an outer surface of the applicator and is a surface capable of receiving a single dose of the preparation and provided for applying (in other words, transferring) the received preparation to the body surface. That is, the therapeutic surface means an area which can be used for spreading the preparation over the intended body surface.

According to one embodiment of the present invention, the "applicator side surface" is an area separate from the therapeutic surface in an outer surface of the applicator. Specifically, the applicator side surface is an area used for holding the applicator by a hand in application and preferably is an area forming a surface extending in a substantially perpendicular direction with respect to the therapeutic surface.

The applicator side surface may have a holding portion for manually operating the applicator, e.g., by the fingertips or hands of a user. Moreover, the applicator side surface may have an undercut for fixing a cover (over-cap) for protecting the therapeutic surface of the applicator.

A preferable therapeutic surface as the therapeutic surface of the applicator according to one embodiment of the present invention is a therapeutic surface formed by a rigid and non-elastic material and having a single recess shape. Here, the "recess" relating to the therapeutic surface means a shape formed to include a center area which is recessed or lower than the periphery so as to receive and hold the pharmaceutical preparation.

The therapeutic surface having the aforementioned shape can be formed by an outer peripheral ridge portion merging into the applicator side surface.

The therapeutic surface and the applicator side surface can merge into a top part of the outer peripheral ridge portion of the therapeutic surface, for example. In this case, the top part of the outer peripheral ridge portion is a substantially circular shape or a substantially oval shape, and the therapeutic surface starts from the top part of the outer peripheral ridge portion toward the inner side in the radial direction applicator surface, and the therapeutic surface extends from the outer peripheral ridge portion, radially spanning the entire width of the applicator.. The top part of the outer peripheral ridge portion can be made into a shape in which a corner or a dent is not generated and as a result, the applicator side surface and the therapeutic surface (specifically, the outer peripheral ridge portion) can merge smoothly into each other.

The top outer surface of the applicator is surrounded by the outer peripheral ridge bounding the therapeutic surface, and can form a single recess or a single flat portion. In either case, the top outer surface of the therapeutic surface and the outer peripheral ridge portion of the therapeutic surface merge smoothly into each other, and a shape in which the whole therapeutic surface is a continuously single recess can be formed.

A preferable therapeutic surface forms a single flat portion. The "single flat portion" means that there are no projections or recesses substantially in the area.

On the therapeutic surface having a recess shape (e.g., a convex or concave surface on at least a portion of the top face), there is a height difference between an uppermost portion of the therapeutic surface (the top part of the outer peripheral ridge portion, for example) and a lowermost portion of the therapeutic surface.

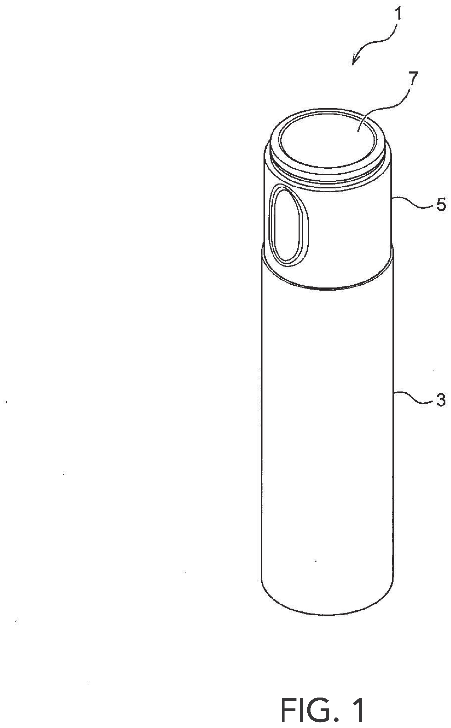

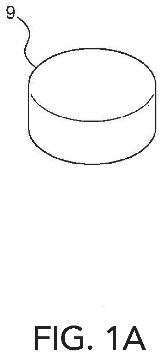

In the specification, terms indicating directions such as "height difference," "upper," "lower" and the like are used in relation with a direction when the applicator is placed so that its therapeutic surface is directed upward as illustrated in Fig. 1 unless specified otherwise.

In one embodiment of the present invention, the height difference between the uppermost portion and the lowermost portion of the therapeutic surface is not particularly limited if it is such a degree that the preparation hardly remains after the application and the liquid drip rarely occurs, but the preferable height difference is 0.1 mm to 4.0 mm. More preferable height difference is 0.1 mm to 1.5 mm. Particularly preferable height difference is 0.2 mm to 0.5 mm.

Moreover, in one embodiment of the present invention, a preferable therapeutic surface is a therapeutic surface in which the therapeutic surface forms the single flat portion and the flat portion occupies 5% or more of the whole area of the therapeutic surface. A more preferable therapeutic surface forms the single flat portion and the flat portion occupies 60% or more of the whole area of the therapeutic surface. The "area of the therapeutic surface" herein means an area in a shape of the therapeutic surface as viewed from above.

Moreover, in one embodiment of the present invention, a preferable therapeutic surface is a therapeutic surface in which a difference in height between the uppermost portion and the lowermost portion of the therapeutic surface is in a range from 0.1 mm to 1.5 mm, the lower portion of the therapeutic surface forms a single flat portion, and the flat portion occupies 5% or more of the whole area of the therapeutic surface.

A more preferable therapeutic surface is a therapeutic surface in which a difference in height between the uppermost portion and the lowermost portion of the therapeutic surface is in a range from 0.1 mm to 1.5 mm, the therapeutic surface forms a single flat portion, and the flat portion occupies 60% or more of the whole area of the therapeutic surface.

Moreover, in one embodiment of the present invention, a preferable therapeutic surface is a therapeutic surface having a substantially circular shape having a diameter in a range from 20 mm to 45 mm or a substantially oval shape having a long or short diameter in a range from 20 mm to 45 mm as viewed from above. A more preferable therapeutic surface is a therapeutic surface having a substantially circular shape having a diameter in a range from 30 mm to 40 mm as viewed from above.

Moreover, in one embodiment of the present invention, a preferable therapeutic surface is a therapeutic surface having a substantially circular shape having a diameter in a range from 20 mm to 45 mm or a substantially oval shape having a long or short diameter in a range from 20 mm to 45 mm as viewed from above and having a height difference within a range from 1/10 to 1/200 of a diameter (or a long diameter or a short diameter). A more preferable therapeutic surface is a therapeutic surface having a substantially circular shape having a diameter in a range from 30 mm to 40 mm as viewed from above and having a height difference within a range from 1/75 to 1/150 of the diameter.

Moreover, in one embodiment of the present invention in which the therapeutic surface merges into the applicator side surface on the top part of the outer peripheral ridge portion and the top of the outer peripheral ridge portion is not flat, if the lower portion of the therapeutic surface forms a single flat portion, a width of the outer peripheral ridge portion as viewed from above (in other words, a horizontal distance to a spot where the lower portion (that is, the flat portion) starts from the top part of the outer peripheral ridge portion toward the inner side in the radial direction) is preferably a length of twice or more of the height difference between the top part of the outer peripheral ridge portion and the flat portion or more preferably a length of three times or more. Particularly preferably it is a length of four times or more.

Moreover, in one embodiment of the present invention, a cover (over-cap) may be attached to the applicator. The cover is attached so as to cover the therapeutic surface of the applicator and is removed in use. The cover is removed prior to administration of the dose of the pharmaceutical preparation and is replaced after the applicator has been used to transfer the dose to the treatment area.

As an example of the "rigid non-elastic material" in one embodiment of the present invention, a polyester resin (polyethylene terephthalate resin, polyacrylate resin), polycarbonate resin, polyethylene resin, polypropylene resin, PVC resin (polyvinylchloride resin) or an epoxy ultraviolet curable resin used for a 3D printer and the like can be cited.

A particularly preferable material as the rigid and non-elastic material is polypropylene resin or a high-density polyethylene resin.

Moreover, in one embodiment of the present invention, a preferable material as the rigid and non-elastic material is a material having tensile strength of 70 kg/cm² to 1760 kg/cm². More preferably, it is a material having tensile strength of 100 kg/cm² to 1500 kg/cm² or particularly preferably a material having tensile strength of 220 kg/cm² to 390 kg/cm²

In the applicator according to one embodiment of the present invention, it is only necessary that at least the therapeutic surface of the applicator is formed by a rigid and non-elastic material. In other words, the material forming a portion other than the therapeutic surface is not particularly limited. Moreover, regarding the applicator, the entirety including the therapeutic surface can be integrally molded as a single component, but a manufacturing method of the applicator is not particularly limited.

The applicator used in the applicator system according to one embodiment of the present invention has a therapeutic surface formed of a rigid and non-elastic material and having a single recess. The rigid therapeutic surface itself is not deformed even if it is pressed onto the body surface during application. Therefore, the preparation can be held on the recess-shaped therapeutic surface during the application, and there is minimal loss of the dose due to spillage or leaking from the therapeutic surface. Moreover, since the therapeutic surface is not deformed, even the preparation having viscosity of a low-to-medium or moderate degree hardly remains on the recess-shaped therapeutic surface after the application. Therefore, the single dose can be reliably transferred to the body surface. Moreover, since the therapeutic surface is not deformed, a sense of discomfort of the user caused by catching of the axilla hair during the application can be also prevented. Moreover, the recess-shaped therapeutic surface does not cause a concern that the preparation drips after receiving or during the application even if it is used for the preparation having viscosity of a low-to-medium or moderate degree, unlike the conventional projecting therapeutic surface. Therefore, the hand operating the applicator or the periphery of the applied portion is not contaminated by the dripping preparation.

Moreover, the applicator according to one embodiment of the present invention can be washed easily and is an applicator with high convenience for a user. Since the preparation hardly remains on the therapeutic surface after the application, the therapeutic surface can be cleaned easily by wiping it with a cotton cloth or the like. Moreover, since there is no projecting/recess surface substantially which would cause liquid collection on the therapeutic surface, the preparation on the therapeutic surface can be removed also by rinsing with water and drying.

The applicator according to one embodiment of the present invention is preferably one such that the preparation received on the therapeutic surface can remain on the therapeutic surface without dripping of the dispensed dose onto the applicator side surface during a certain period of time. The "certain period of time" refers to time after the preparation is received by the therapeutic surface of the applicator until the application on the body surface is started. Specifically, it is at least 3 seconds, preferably 10 seconds, more preferably 20 seconds, further preferably 30 seconds, and particularly preferably 60 seconds.

The preparation suitable for the applicator and its applicator system according to one embodiment of the present invention is not particularly limited as long as it is a pharmaceutical preparation for topical or transdermal administration having viscosity of a low-to-medium or moderate degree and applied on the body surface, but it is preferably a hyperhidrosis therapeutic agent applied on the axilla, for example. As the hyperhidrosis therapeutic agent, a preparation containing sofpironium bromide described above is preferable.

Each embodiment of the present invention will be described below by referring to the attached drawings. The following explanation merely illustrates an example and is not intended to limit a technical range of the invention of the present application to the following embodiments. Moreover, in the figures, the same reference numerals are given to the same or corresponding constituent elements, and duplicated explanation will be omitted.

Fig. 1 is a perspective view of an applicator system 1 according to one embodiment of the present invention. The applicator system 1 includes a container 3 and an applicator 5. The container 3 is configured to accommodate and hold and store a pharmaceutical preparation for topical or transdermal administration. The pharmaceutical preparation preferably has viscosity of a low-to-medium or moderate degree from 100 to 2000 centipoise at 25°C. The applicator system 1 may include a cover (in other words, an over-cap) member 9 covering a therapeutic surface (which will be described later) of the applicator 5 as illustrated in Fig. 1A.

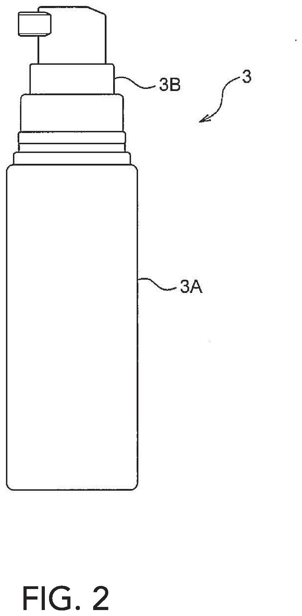

The applicator 5 is configured to be detachably attached to the container 3. Fig. 2 illustrates a side elevational view of the applicator system of the invention, showing the container 3 having attached thereto a dispenser 3B when the applicator is separated from the container 3. In the example in Fig. 2, the container 3 includes a bottle portion 3A for accommodating the pharmaceutical preparation and a pump portion or dispenser 3B attached to a mouth part (reference numeral omitted) of the bottle portion 3A. The pharmaceutical preparation accommodated in the bottle portion 3A is supplied to the applicator 5 by the pump portion 3B. However, the container 3 does not necessarily have to include the pump portion 3B. In this case, the pharmaceutical preparation may be supplied directly to the applicator 7 from the mouth part of the bottle portion 3A.

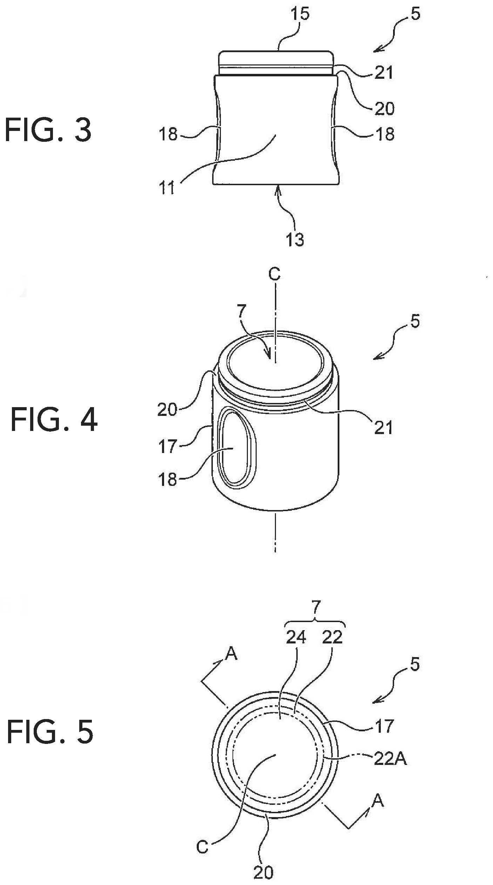

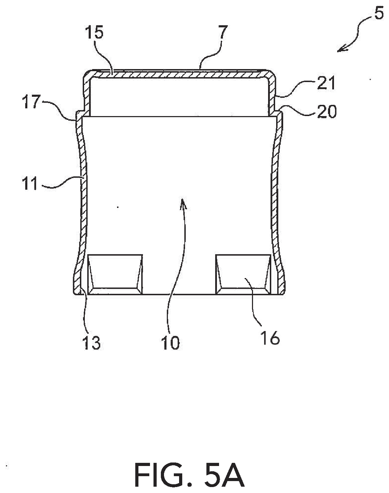

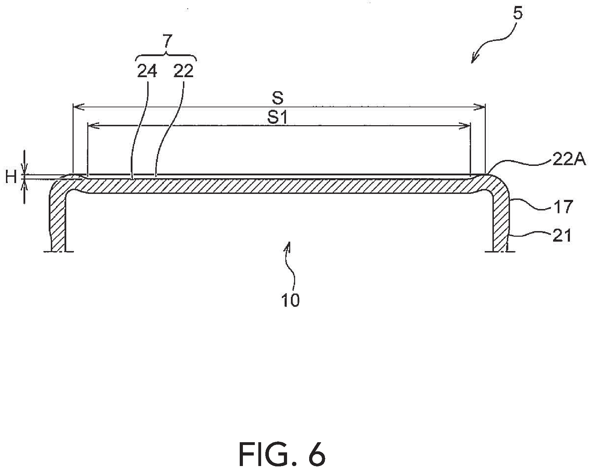

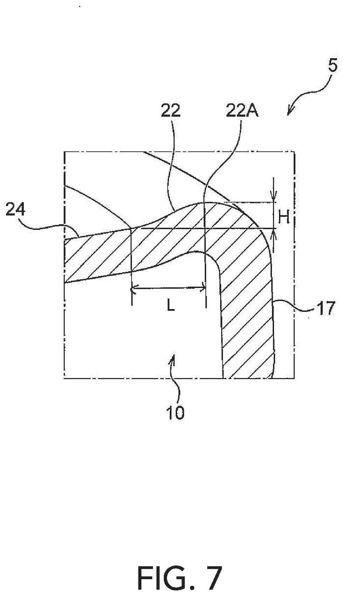

Subsequently, the configuration of the applicator 5 will be specifically explained. Figs. 3 to 5 are a side view, a top perspective view, and a top plan view of the applicator 5 illustrated at Fig. 1, respectively. Figs. 5A and 6 are side sectional views including partially enlarged side sectional view of the applicator 5 along an A-A line in Fig. 5. Fig. 7 is a perspective sectional view illustrating a part of the therapeutic surface 7 of the applicator in an enlarged manner.

Fig. 5A also illustrates an inner surface of the side wall comprising one or more protrusions 16 for matingly engaging with a top portion of the container or a portion of the dispenser provided with the container. The embodiment illustrated shows two protrusions in the cross-sectional view, and thus providing a total of four protrusions around the circumference of the circular applicator. It would be understood that the protrusions can be formed integrally with the applicator side wall by the molding process or can be added separately to the applicator side wall. The protrusions can be formed as a single peripheral flange around the entire circumference of the applicator inner surface, or can be provided as discrete projections spaced intermittently, preferably equidistant from one another. When formed as a single flange, the flange can be planar forming a singular ring around the inner surface or may be spiraled forming threads to matingly engage a threaded top portion of the container or dispenser portion.

Additionally, it would be understood that the two or more discrete protrusions can be provided, preferably three or more, and more preferably numbering at least four and more preferably, eight, protrusions. Each protrusion can be the same size or they can be different sizes and different shapes as desired. Applicants have discovered that eight protrusions spaced equidistant around the periphery of the bottom, inner edge of the inner surface of the side wall of the applicator provides a secure engagement with the container, while allowing for easy attachment and removal of the applicator and increasing the efficiency and efficacy of the seal to prevent drying and evaporation of the composition within the container, and which further can provide a preferred seating of the applicator onto the container which is more aesthetically and commercially pleasing and desirable.

Referring to Fig. 3, the applicator 5 includes a substantially cylindrical body 11 having a closed upper end portion 15 and an open lower end portion 13. The applicator 5 has an internal space or cavity open through the lower end portion 13 of the body 11 so as to receive the mouth part (a dispensing part of the pump portion 3B in the illustrated example) of the container 3 (see Fig. 5A, for example). As a result, the applicator 5 can be attached to the container 3 so as to cover the mouth part (including the dispensing part of the pump portion 3B in the illustrated example) of the container 3.

An outer surface of the closed upper end portion 15 of the body 11 includes the therapeutic surface 7 (shown in FIG. 4). As described above, the "therapeutic surface" is the area in a certain range of the applicator and means a surface which can receive a single dose of the pharmaceutical preparation and can be used for spreading the received pharmaceutical preparation over the body surface of the user. The therapeutic surface 7 has a single recess. The "recess" relating to the therapeutic surface means a shape formed with a height lower than the periphery so as to receive the pharmaceutical preparation.

As further illustrated in FIGs. 4 and 5, the body 11 of the applicator 5 includes a side surface 17. The side surface 17 of the applicator 5 is an area used by the user for holding the applicator 5 by the hand in use of the applicator 5 and is an area separate from the therapeutic surface 7. The side surface 17 preferably extends to a substantially perpendicular direction to the therapeutic surface 7. However, the direction in which the side surface 17 extends does not necessarily have to be a direction perpendicular to the therapeutic surface 7.

For example, as illustrated in Figs. 3 and 4, the side surface 17 of the applicator 5 can include a pair of recess holding portion 18 at positions faced in a radial direction of the applicator 5, for example (only one of the holding portions 18 is illustrated in Fig. 4). The user can hold the holding portion 18 by the fingertips when the applicator 5 is separated from the container 3 for use.

Moreover, in an embodiment referenced in FIGs. 3-5, the applicator 5 has a shoulder portion 20 for receiving a cover member 9. Moreover, the side surface 17 of the applicator 5 has a gradient portion (in other words, an undercut) 21 slightly inclined downward to the inner side on an upper part of the shoulder portion 20. The gradient portion 21 can act as a locking part for locking the cover member 9 by the applicator 5. As a result, when the applicator system 1 is not in use, the cover member 9 (FIG. 1A) can be affixed to the applicator 5 so as to protect the therapeutic surface 7.

As illustrated in Figs. 6 and 7, the therapeutic surface 7 of this embodiment is defined by the outer peripheral ridge portion 22 merging into the side surface 17 of the applicator 5 and the top outer surface of "therapeutic surface" 24 surrounded by the outer peripheral ridge portion 22. The therapeutic surface 7 and the side surface 17 of the applicator 5 merge into each other on a top part 22A of the outer peripheral ridge portion 22. Therefore, the therapeutic surface 7 forms the outer peripheral ridge portion 22 and the lower portion 24 in order from the top part 22A of the outer peripheral ridge portion 22 toward the inner side in the radial direction. The top part 22A of the outer peripheral ridge portion 22 has a shape in which a corner or a dent is not generated and as a result, the side surface 17 of the applicator 5 and the therapeutic surface 7 (specifically, the outer peripheral ridge portion 22) merge smoothly into each other.

In this embodiment, as illustrated in the plan view of Fig. 5, the therapeutic surface 7 has a substantially circular shape as viewed from above around a center axis C of the body 11 of the applicator 5 in a length direction. The diameter of the circular therapeutic surface 7 can be set in a range from 20 mm to 45 mm, for example. The more preferable therapeutic surface 7 is a therapeutic surface having a substantially circular shape with the diameter from 30 mm to 40 mm as viewed from above. However, in another embodiment, the therapeutic surface 7 may have a substantially oval shape as viewed from above around the center axis C of the body 11 of the applicator 5. In this case, a long or short diameter of the therapeutic surface 7 can be set in a range from 20 mm to 45 mm, for example.

In the embodiment, the lower portion 24 forms a single flat portion. As illustrated in Figs. 6 and 7, the lower portion 24 and the outer peripheral ridge portion 22 merge smoothly into each other, and the entire therapeutic surface 7 forms a shape which is continuously (in other words, including no projection) single recess shape.

An area of the lower portion 24 (an area of a region indicated by S1 in Fig. 6) forming the flat portion preferably occupies 5% or more of the whole area (an area of a region defined by the top part 22A and indicated by S in Fig. 6) of the therapeutic surface 7. The lower portion 24 forming the flat portion more preferably occupies 60% or more of the whole area of the therapeutic surface 7.

The whole area of the therapeutic surface and the area of the lower portion (i.e. S, S1 and the like) in the specification is the area as viewed from above.

As illustrated in Figs. 6 and 7, there is a height difference H between an uppermost portion of the therapeutic surface 7 (the top part 22A of the outer peripheral ridge portion 22 in this embodiment) and a lowermost portion of the therapeutic surface 22 (the flat surface forming the lower portion 24 in this embodiment). The height difference H between the top part 22A in the outer peripheral ridge portion 22 of the therapeutic surface 7 and the lowermost portion 24 is not particularly limited as long as it has such a dimension that the pharmaceutical preparation received by the therapeutic surface 7 can be applied on the body surface with hardly any remaining on the therapeutic surface 7 after application and liquid dripping rarely occurs, but the preferable height difference H is from 0.1 mm to 4.0 mm. The more preferable height difference H is from 0.1 mm to 1.5 mm. The particularly preferable height difference H is from 0.2 mm to 0.5 mm.

Moreover, in one embodiment of the present invention, the preferable therapeutic surface 7 is a therapeutic surface in which the height difference H between the uppermost portion and the lowermost portion of the therapeutic surface 7 is in a range from 0.1 mm to 1.5 mm, the lower portion 24 of the therapeutic surface 7 forms the single flat portion, and the flat portion occupies 5% or more of the whole area of the therapeutic surface 7.

The more preferable therapeutic surface is a therapeutic surface in which the height difference H between the uppermost portion and the lowermost portion of the therapeutic surface 7 is in a range from 0.1 mm to 1.5 mm, the lower portion 24 of the therapeutic surface 7 forms the single flat portion, and the flat portion occupies 60% or more of the whole area of the therapeutic surface 7.

Moreover, in one embodiment of the present invention, the preferable therapeutic surface 7 is a therapeutic surface having a substantially circular shape having a diameter in a range from 20 mm to 45 mm as viewed from above or a substantially oval shape having a long or short diameter in a range from 20 mm to 45 mm as viewed from above and having a height difference H within a range from 1/10 to 1/200 of a diameter (or a long diameter or a short diameter). The more preferable therapeutic surface 7 is a therapeutic surface having a substantially circular shape when viewed from above and having a diameter in a range from 30 mm to 40 mm and having a height difference H within a range from 1/75 to 1/150 of the diameter.

Moreover, in one embodiment of the present invention, a width of the outer peripheral ridge portion as viewed from above (a horizontal distance L to a spot where the lower portion (that is, the flat portion) 24 starts from the top part 22A of the outer peripheral ridge portion 22 toward the inner side in the radial direction in the illustrated example) (see Fig. 7) is preferably a length of twice or more of the height difference between the top part of the outer peripheral ridge portion and the flat portion or more preferably a length of three times or more. Particularly preferably it is a length of four times or more.

The lower outer portion 24 does not necessarily have to form the flat portion. The top outer surface 24 may form a curved recess surface portion continuously curved with a certain radius of curvature from the top part 22A of the outer peripheral ridge portion 22 toward the center axis C, for example. In this case, a center of the recess surface portion becomes the lowermost portion of the therapeutic surface 7, for example.

In Fig.6 the top part 22A of the outer peripheral ridge portion 22 of the therapeutic surface 7 is the uppermost portion, the flat area of the top outer surface 24 is the lowermost portion, and the height difference is 0.38 mm. A volume of the therapeutic surface of the applicator in Fig. 6 is 0.29 mL.

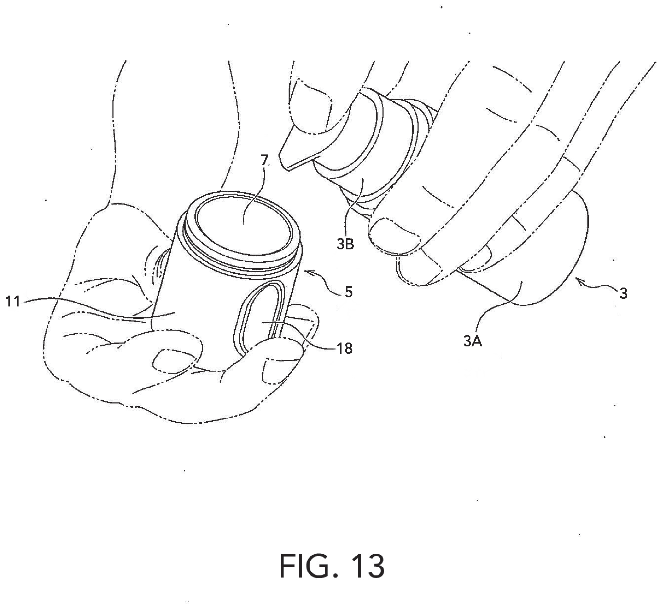

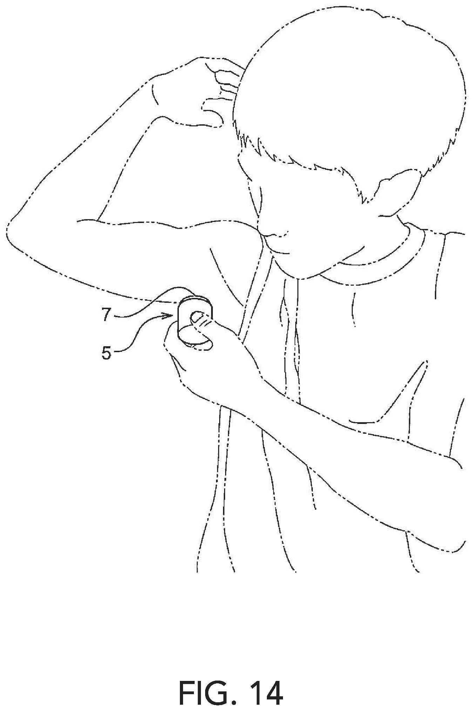

The applicator 5 according to one embodiment is used in a state separated from the container 3. Specifically, the cover member 9 is removed from the applicator 5, and the applicator 5 is removed from the container 3. After that, as illustrated in Fig. 13, for example, the fingertips of one of the hands are placed on the holding portion 18, and the body 11 is held so as to be sandwiched, while the pump portion 3B of the container 3 is operated by the other hand, and a single dose of the pharmaceutical preparation is dispensed on the therapeutic surface 7. After that, as illustrated in Fig. 14, for example, the pharmaceutical preparation is spread over the skin surface while the therapeutic surface 7 is pressed onto the axilla.

Since the therapeutic surface 7 is formed by a rigid non-elastic material, even if it is pressed onto the body surface during the application, it is not deformed. Therefore, the preparation can be held in the therapeutic surface 7 having a recess shape also during the application, and there is hard that the preparation leaks out of the therapeutic surface 7. Moreover, since the therapeutic surface 7 is not deformed, even the preparation with viscosity of a low-to-medium or moderate degree hardly remains on the recess-shaped therapeutic surface 7 after the application. Therefore, the single dose can be transferred reliably to the skin. Moreover, since the therapeutic surface 7 is not deformed, a sense of discomfort of the user caused by catching of the axilla hair during the application can be also prevented. Moreover, the recess-shaped therapeutic surface 7 does not cause a concern that the preparation drips after placement of the dose on the therapeutic surface or during the application even if it is used for the preparation having viscosity of a low-to-medium or moderate degree, unlike the conventional projecting therapeutic surface. Therefore, the hand operating the applicator 5 or the periphery of the applied portion is not contaminated by the dripping preparation and used adequately.

Moreover, the applicator 5 can be washed easily. Since the preparation hardly remains on the therapeutic surface 7 after the application, the therapeutic surface 7 can be cleaned easily by wiping with a cotton cloth or the like. Moreover, since there are no projections or recesses that could collect a liquid on the therapeutic surface 7, the preparation on the therapeutic surface 7 can be removed also by rinsing, and drying.

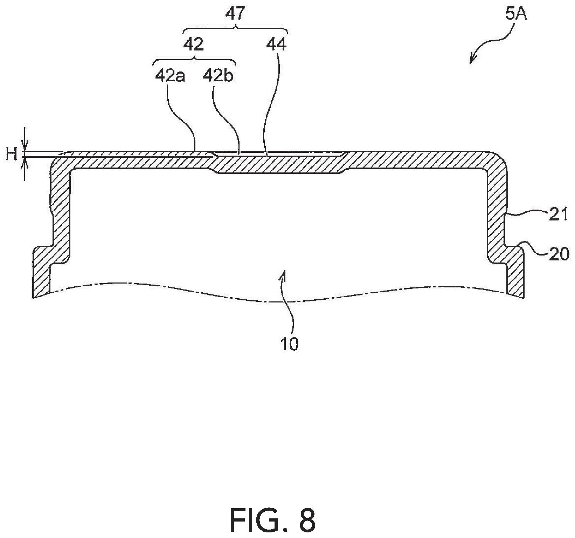

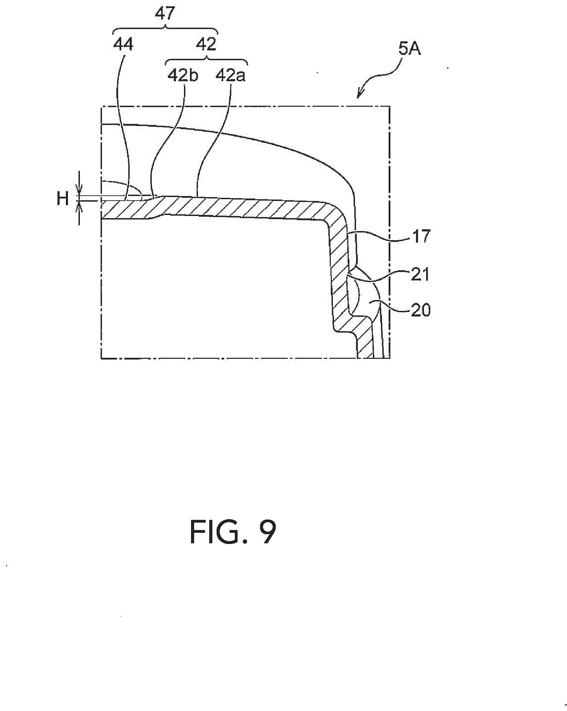

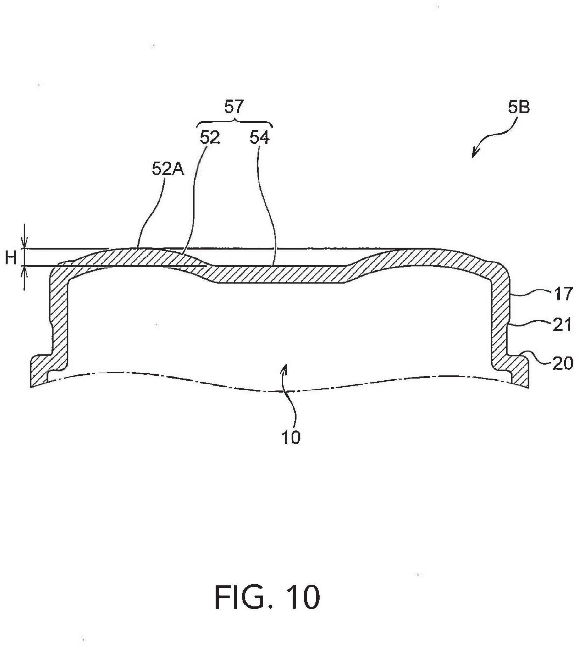

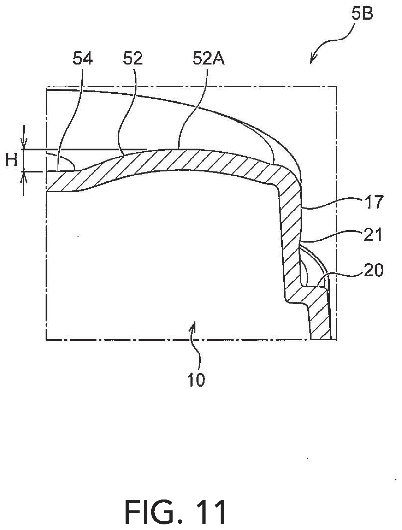

Figs. 8 and 9 are views corresponding to Figs. 6 and 7, respectively, illustrating a first variation of the embodiment. Figs. 10 and 11 are views corresponding to Figs. 6 and 7, respectively, illustrating a second variation of the embodiment.

In an applicator 5A of a first variation, a therapeutic surface 47 has an outer peripheral ridge portion 42 and a top outer surface 44 surrounded by the outer peripheral ridge portion 42. The top outer surface 44 forms a flat portion. In the illustrated example, the top outer surface 44 has a circular shape having a diameter of approximately one third of a diameter of the therapeutic surface 47. The outer peripheral ridge portion 42 has a transfer portion 42b to the top outer surface 44 and a substantially flat portion 42a. In this case, the flat portion 42a of the outer peripheral ridge portion 42 is the uppermost portion of the therapeutic surface 47 and the flat surface forming the top outer surface 44 is the lowermost portion.

In an applicator 5B of a second variation, a therapeutic surface 57 has an outer peripheral ridge portion 52 and a top outer surface 54 surrounded by the outer peripheral ridge portion 52. The top outer surface 54 forms a flat portion. Unlike the first variation, the outer peripheral ridge portion 52 has a shape of a gentle annular raised portion formed between the side surface 17 of the applicator 5B and the top outer surface 54. This raised portion does not have a corner part but is smooth. In this example, a top part of the raised portion is the uppermost portion of the therapeutic surface 57 and the flat surface forming the top outer surface 54 is the lowermost portion.

In the second variation, the top outer surface 54 does not necessarily have to form the flat surface. For example, the top outer surface 54 may form a recess surface portion continuously curved from the transfer portion to the outer peripheral ridge portion 52 toward the center axis C. In this case, the center of the recess surface portion is the lowermost portion of the therapeutic surface 57.

### EXAMPLES

The present invention will be described below more specifically by citing an example.

In compliance with the known methods, various preparations having viscosity of a low-to-medium or moderate degree were prepared.

The viscosity was measured under the conditions shown below by using a conical-planar rotary viscometer, RE550 Viscometer by Toki Sangyo Co., Ltd.

**TABLE 1**

| **Components** | **Composition 1 (w/w%)** | **Composition 2-1 (w/w%)** | **Composition 2-2 (w/w%)** |
|---|---|---|---|
| Sofpironium Bromide | 15 | 0 | 0 |
| HPC | 1.25^{*1} | 1.25^{*1} | 1.25^{*2} |
| Other additives | 12.55 | 12.55 | 12.55 |
| Absolute ethanol | 71.20 | 86.20 | 86.20 |
| viscosity (centipoise at 25°C) | 824 | 626 | 111 |

| | | | |
|---|---|---|---|
| *1: Klucel MF by Ashland Co., Ltd. *2: HPC-H by Nippon Soda Co., Ltd | | | |

Viscosity measurement conditions are presented in TABLE 2, below:

**TABLE 2 - Measurement conditions**

| | |
|---|---|
| Measurement temperature | 25°C |
| Preheating time | 30 seconds |
| Measurement sample | 1 mL |
| Cone rotor (R-H1°34'×R24) | angle:1°34', radius:24mm |
| Rotation speed | 5 rpm |

The following test Example 1 and test Example 2 were conducted by using these preparations. In each of the test Examples, the applicator in one embodiment described in Fig. 1 and detailed in Fig.3 to Fig. 7 was used.

### TEST EXAMPLE 1

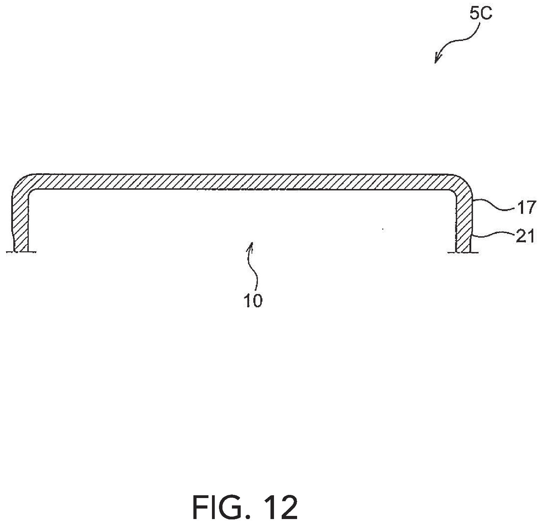

The aforementioned applicator was placed still with the therapeutic surface faced up, a single dose (0.65 mL) of the formulation of Composition 1 was dripped at a spot at a distance of 10 mm from the center of the therapeutic surface, and time (seconds) until the preparation flows down to the side surface of the applicator was counted. If the preparation did not drip to the applicator side surface even after 60 seconds elapsed, it was determined that the preparation was held by the applicator, and the test was finished. The test was conducted repeatedly five times, and if there was no drip to the applicator side surface in the five sessions of the test, it was determined to be "A", and if there was a drip to the applicator side surface only in one of the five sessions, it was determined to be "B". Moreover, the similar test was conducted by using the formulations of Composition 2-1 and Composition 2-2. Moreover, as a comparative example of the present invention, a similar test was conducted by using an applicator 5C (its therapeutic surface has a circular shape with a diameter of 33 mm as viewed from above) having the therapeutic surface formed only by a flat surface illustrated in Fig. 12.

The result is shown in Table 3.

**TABLE 3**

| Applicator | Composition | First session | Second session | Third session | Fourth session | Fifth session | Determination |
|---|---|---|---|---|---|---|---|
| | Viscosity (centipoise (25°C)) | Time until formulation drips to applicator side surface (seconds) | | | | | |
| Applicator with recess surface*1 | Composition 1 824 | >60 | >60 | >60 | >60 | >60 | A |
| Applicator with flat surface*2 | | 4 | >60 | >60 | 12 | >60 | B |
| Applicator with recess surface*1 | Composition 2-1 626 | >60 | >60 | >60 | >60 | >60 | A |
| Applicator with flat surface*2 | | >60 | >60 | 6 | >60 | 7 | B |
| Applicator with recess surface*1 | Composition 2-2 111 | >60 | >60 | >60 | >60 | >60 | A |
| Applicator with flat surface*2 | | >60 | >60 | 4 | 3 | >60 | B |

| | | | | | | | |
|---|---|---|---|---|---|---|---|
| *1: Applicator of the present invention described in Fig. 1 *2: Applicator of the comparative example described in Fig. 12 | | | | | | | |

From the result of the test Example 1, it was made clear that the applicator according to one embodiment of the present invention has high capability of the therapeutic surface for holding the preparation as compared with the applicator with the flat surface and can apply the preparation on the desired body surface without liquid drip for a certain period of time after the applicator receives the preparation.

### TEST EXAMPLE 2

For each of Composition 2-1 and Composition 2-2, a single dose (0.65 mL) was dispensed to the center of the therapeutic surface in the applicator, and it was applied to the axilla. By measuring a weight of the applicator after the application and taking a difference from a tare weight of the applicator, an amount of the preparation remaining on the therapeutic surface after the application and the average remaining rate to a single dose was calculated.

For each of the applicator to which the present invention was applied (the applicator with the recess surface described in Fig. 1) and the applicator having a flat therapeutic surface (the comparative example, the applicator with the flat surface described in Fig. 12), application was carried out repeatedly three times for each of three test subjects, and average values of remaining amounts rate remaining on the applicators were calculated. The applicator (recess surface) to which the present invention was applied had 6.1% (Composition 2-1) to 7.5% (Composition 2-2) of the preparation remaining on the therapeutic surface in the single dose, while in the comparative example, 5.6% (Composition 2-1) to 8.9% (Composition 2-2) of the preparation remaining for the single dose. No significant difference is found between the both. That is, it is found that the applicator described in Fig. 1 has the preparation remaining amount of a degree as small as that of the applicator in the comparative example having the therapeutic surface formed only by the flat surface.

From the results in the test Example 1 and the test Example 2, it was found that the applicator of the applicator system according to one embodiment of the present invention can hold the preparation having viscosity of a low-to-medium or moderate degree without causing liquid drip and can reduce the preparation remaining amount after the application. That is, the applicator according to one embodiment of the present invention has proved to be useful as an applicator of a pharmaceutical preparation having viscosity of a low-to-medium or moderate degree for topical or transdermal administration.

### INDUSTRIAL APPLICABILITY

The applicator system according to one embodiment of the present invention can be applied to an applicator system for a pharmaceutical preparation for topical or transdermal administration with viscosity of a low-to-medium or moderate degree.

## Claims

1. An applicator (5) in the form of a cap for applying a pharmaceutical preparation comprising sofpironium bromide, in an amount effective for treating or ameliorating excessive sweating or hyperhidrosis, to a skin surface of a patient in need thereof, wherein the pharmaceutical preparation is in the form of a gel, lotion, ointment, or cream having a moderate viscosity of from 100 centipoise to 2,000 centipoise (0.1 to 2 Pa.s) at 25°C, said applicator in the form of a cap comprising:
one or more substantially rigid side walls (17) bounding a cavity which is open at a bottom end (13) and closed at a top end (15) by a top wall substantially perpendicular to said one or more substantially rigid side walls to form the applicator in the form of a cap which fits over and encloses a top portion of a container or dispenser (3) for the pharmaceutical preparation, the substantially rigid side walls being configured to detachably and matingly engage with the top portion of the container or dispenser to form an air-tight seal between the applicator in the form of a cap and the container or dispenser, said closed top end of the applicator in the form of a cap having an outer surface comprising:
a central flat, rigid, and continuous face (7) which is solid and non-porous such that liquid cannot pass through said central flat, rigid, and continuous face, the central flat, rigid, and continuous face being useful for receiving a single dose of 0.1 mL to 1.5 mL of the pharmaceutical preparation dispensed thereon from the container or dispenser, and
a rigid peripheral ridge (22, 42, 52) extending up from the outer surface of said closed top end, the rigid peripheral ridge bounding the central flat, rigid, and continuous face and forming a reservoir area for retaining the pharmaceutical preparation within said reservoir area when the pharmaceutical preparation is dispensed thereon from the container or dispenser,
wherein the applicator in the form of a cap is integrally molded as a single component;
wherein the difference in height between an uppermost portion of the rigid peripheral ridge and the central flat, rigid and continuous face ranges from 0.1 mm to 1.5 mm, wherein the top edge of the rigid peripheral ridge has a rounded surface to form an application surface, wherein the width of the top part of the rigid peripheral ridge is twice or more of the difference in height between an uppermost portion of the rigid peripheral ridge and the central flat, rigid and continuous face,
wherein the one or more substantially rigid side walls comprise indentations (18) to facilitate gripping and handling or attaching or detaching the applicator in the form of a cap; and
wherein the inner surface of the one or more substantially rigid side walls comprises one or more ridges or protrusions formed thereon (16).

2. The applicator in the form of a cap of claim 1, wherein the ridges or protrusions on the inner surface of the one or more substantially rigid side walls matingly engage with the top portion of the container or dispenser to detachably affix the applicator in the form of a cap to the container or dispenser.

3. The applicator in the form of a cap of claim 1, further comprising an overcap (9) which covers at least the central flat, rigid and continuous face of the applicator in the form of a cap.

4. An applicator system for applying a pharmaceutical preparation comprising sofpironium bromide effective for treating or ameliorating excessive sweating or hyperhidrosis, wherein the pharmaceutical preparation is in the form of a gel, lotion, ointment, or cream having a moderate viscosity of from 100 centipoise to 2,000 centipoise (0.1 to 2 Pa.s) at 25°C, to a skin surface of a patient in need thereof, said system comprising:
- a container or dispenser for housing and storing a plurality of doses of the pharmaceutical preparation, and
- an applicator (5) in the form of a cap for applying the pharmaceutical preparation to a skin surface of a patient in need thereof, said cap comprising:
one or more substantially rigid side walls (17) bounding a cavity which is open at a bottom end (13) and closed at a top end (15) by a top wall substantially perpendicular to said one or more substantially rigid side walls to form the applicator in the form of a cap which fits over and encloses a top portion of the container or dispenser (3) for the pharmaceutical preparation, the substantially rigid side walls being configured to detachably and matingly engage with the top portion of the container or dispenser to form an air-tight seal between the applicator in the form of a cap and the container or dispenser, said closed top end of the applicator in the form of a cap having an outer surface comprising:
a central flat, rigid and continuous face (7) which is solid and non-porous such that liquid cannot pass through said central flat, rigid and continuous face, the central flat, rigid and continuous flat outer face being useful for receiving a single dose of 0.1 mL to 1.5 mL of the pharmaceutical preparation dispensed thereon from the container or dispenser, and
a rigid peripheral ridge (22, 42, 52) extending up from the outer surface of said closed top end, the rigid peripheral ridge bounding the central flat, rigid and continuous face and forming a reservoir area for retaining the pharmaceutical preparation within said reservoir area when the pharmaceutical preparation is dispensed thereon from the container or dispenser,
wherein the applicator in the form of a cap is integrally molded as a single component;
wherein the difference in height between an uppermost portion of the peripheral ridge and the central flat, rigid and continuous face ranges from 0.1 mm to 1.5 mm, wherein the top edge of the rigid peripheral ridge has a rounded surface to form an application surface,
wherein the width of the top part of the rigid peripheral ridge is twice or more of the difference in height between an uppermost portion of the rigid peripheral ridge and the central flat, rigid and continuous face,
wherein the one or more substantially rigid side walls comprise indentations (18) to facilitate gripping and handling or attaching or detaching the applicator in the form of a cap; and
wherein the inner surface of the one or more substantially rigid side walls comprises one or more ridges or protrusions formed thereon (16);
the applicator in the form of a cap optionally comprising an overcap (9) which covers at least the reservoir area on the central rigid, flat and continuous face.

5. The applicator system of claim 4, wherein the ridges or protrusions on the inner surface of the one or more substantially rigid side walls matingly engage with the top portion of the container or dispenser to detachably affix the applicator in the form of a cap to the container or dispenser.

6. The applicator system of claim 4, wherein the container or dispenser is a metered-dose pump dispenser.

7. The applicator system of claim 5, wherein the metered-dose pump dispenser dispenses a single dose in a range from 0.1 mL to 1 mL; optionally 0.5 mL to 0.8; optionally 0.55 mL to 0.75 mL.

8. Use of the applicator in the form of a cap of claim 1 or the applicator system of claim 4 in a method for treating or ameliorating excessive sweating or hyperhidrosis in a patient in need thereof, said method comprising the steps of.
a) removing the applicator in the form of a cap from the container or dispenser and, if present, removing the overcap from the applicator in the form of a cap;
b) dispensing one or more doses of the pharmaceutical preparation into the reservoir area on the central rigid, flat and continuous face of the applicator in the form of a cap; and
c) applying the dispensed dose or doses of the pharmaceutical preparation onto the skin surface of the patient.

9. The use of claim 8, wherein the ridges or protrusions on the inner surface of the one or more substantially rigid side walls matingly engage with the top portion of the container or dispenser to detachably affix the applicator in the form of a cap to the container or dispenser.

10. The use of claim 8, wherein the container or dispenser is a metered-dose pump dispenser.

11. The use of claim 10, wherein the metered-dose pump dispenser dispenses a single dose in a range from 0.1 mL to 1 mL; optionally 0.5 mL to 0.8; optionally 0.55 mL to 0.75 mL.
